# EUROPEAN PATENT SPECIFICATION

(11) **EP 2 583 658 B1**
(45) Date of publication and mention of the grant of the patent: **06.05.2020**
(21) Application number: 11795161.6
(22) Date of filing: 09.06.2011
(51) Int. Cl.: A61H 19/00, A61F 5/41, A61N 5/06, A61F 7/00

(54) **SYSTEM FOR ENLARGING AND EXERCISING PENIS**
SYSTEM ZUR VERGRÖSSERUNG UND ZUM TRAINING DES MÄNNLICHEN GESCHLECHTSTEILS
SYSTÈME PERMETTANT D'AGRANDIR ET D'EXERCER LE PÉNIS

(30) Priority: 18.06.2010 CN 201020245046 U
(43) Date of publication of application: 24.04.2013
(73) Proprietor: Li, Hui, Yangzhou, Jiangsu 225001 (CN)
(72) Inventor: Li, Hui, Yangzhou, Jiangsu 225001 (CN)
(74) Representative: Petraz, Gilberto Luigi
(86) International application number: PCT/CN2011/075498
(87) International publication number: WO 2011/157195

(56) References cited:
- EP-A2- 0 131 305
- WO-A1-2007/000013
- CN-A- 101 433 494
- CN-A- 101 433 494
- CN-Y- 201 135 590
- CN-Y- 201 375 651
- CN-Y- 201 418 807
- DE-C1- 4 310 981
- US-A- 4 967 738
- US-A- 4 967 738
- US-A1- 2007 179 337
- US-A1- 2009 099 413
- US-A1- 2009 105 529
- US-B1- 6 338 721

## Description

### FIELD OF THE INVENTION

The present invention relates to a penis enlargement exercising system, and more particularly to an exercising device to enlarge the penis by massage exercising.

### BACKGROUND OF THE INVENTION

Now, penis enlargement machines for massaging and enlarging the penis include a vacuum stretcher and a penis stretcher, and are disclosed by US2009/0247377 A1 and US7,276,040 B2. The penis enlargement machines use a part to hold the glans and pull the glans away from the bottom of the penis to enlarge the penis. But, these products have serious problems, for the over pulling force pains the vulnerable penis, and the insufficiency pulling force is ineffective. Thus, the effects of these products based on the pulling method mentioned above are low.

In 2007, the applicant of the present application filed a China patent application No. 2007101864311, which discloses a penis enlargement exercising system, and some samples are fabricated to test the design, wherein the applicant found that the embodiments 1, 2 and 3 need the hand of a user to overcome the elastic resistance of the rollers to press the penis. The hand got tired quickly and thus it is difficult to operate. As to the embodiment 4, the prepuce may be pushed into the gap formed between the rollers and even clamped by the rollers. These problems are unexpected in the designing stage.

As a result, it is necessary to provide a penis massage exercising system to solve the problems existing in the conventional technologies, as described above. CN201135590Y, according to the preamble of claim 1, is also filed by the applicant of the present application filed on November 18, 2007, and substantially the same as the CN2007101864311, wherein the rollers 18, 25, 22 and 28 of CN201135590Y form a hole for pressing the penis, instead of the frame 7 and the sliders 20. Therefore, a user's hand needs to overcome the elastic resistance of the rollers to press the penis. The hand got tired quickly and thus it is difficult to operate. Moreover, the prepuce may be pushed into the gap formed between the rollers and even clamped and damaged by the rollers.

### SUMMARY OF THE INVENTION

A primary object of the present invention is to provide a penis enlargement exercising system to enlarge the penis of a user both in the circumference and the length thereof, and make the penis healthy by massaging the penis through using the penis enlargement exercising system for a male.

To achieve the above object, the present invention provides a penis enlargement exercising system according to claim 1.

The penis enlargement exercising system
includes a penis enlargement exerciser, a penis protecting cover, a heating device, and a pubic hair separating plate, etc.

The object of the present invention are realized as below: the penis enlargement exerciser of the penis enlargement exercising system comprises a rigid frame and at least one slider, the slider can slide on the frame back and forth; the frame and the slider commonly form a closed circle around a penis of a user; the closed circle is similar to the cross-section of the penis; the circle has no gap in its circumferential direction. When using the penis enlargement exerciser, the penis is put into the closed circle, the slider is forced to move toward an axis of the penis to push the slider toward a center of the penis, and then the closed circle is narrowed, so as to cause the penis to be held tightly by the slider and the frame. The axis of the penis is vertical or substantially vertical to the closed circle. When moving the penis enlargement exerciser, the closed circle is tightly kept around the penis, and the closed circle will slide back and forth on the penis in the direction of the axis of the penis, so that the blood inside the penis is pressed to enlarge a front part of a cavernous body of the penis in the moving direction of the penis enlargement exerciser. The slider can slide on the frame through a shaft, a hole or a groove, or the slider can also rotate on the frame along an axis of the frame, which causes the slippage between the slider and the frame.

To improve the blood circulation in the penis, before using the penis enlargement exerciser, a penis heating device can be used to warm up the penis. So the penis enlargement exercising system further includes a penis heating device which has tube shaped or half-tube shaped heating material put around the penis. It is similar to the shape of the penis and has a cross-section area similar to the size of the penis but slightly greater than that of the penis. The penis heating element can be made of material which can generate far infrared rays, and includes a heating control circuit and components thereof. When using, the penis will be put into the tube shaped penis heating device which connects with a power source, and then the far infrared rays radiated by the heating device will cover and warm up the penis.

The penis enlargement exercising system further includes a penis protecting cover, which is tube shaped or half tube shaped, to protect the prepuce (the skin of the penis) from being pushed into a gap between the slider and the frame. The penis protecting cover is put around the penis and will separate the penis from the gap between the slider and the frame. To avoid from being pushed into the gap, the thickness of the penis protecting cover over the gap is preferably not smaller than 0.25 mm. The penis protecting cover may be made of soft material, such as silicon gel, rubber, and latex, etc.

To avoid the pubic hair being pulled by the exerciser, a pubic hair separating plate is further included, and a hole is formed on the center of the pubic hair separating plate, and the size of the hole is similar to the cross-section of the penis. A portion around the hole is made of soft material.

Preferably, the frame has at least one U-shaped hard part which has two shafts parallel to each other, and two holes parallel to each other are formed on the slider. The shafts of the U-shaped part pass through the two holes, respectively.

Preferably, the frame has at least two shafts parallel to each other, and two holes parallel to each other are formed on the slider. The two shafts pass through the two holes, respectively. The slider can slide on the shafts.

Preferably, the penis enlargement exerciser has at least two of the sliders. The sliders are put around the penis.

Preferably, the penis enlargement exerciser has at least two of the sliders. There are two holes parallel to each other on each of the sliders. The frame has at least two shafts parallel to each other. The shafts pass through the holes on the sliders, respectively. The sliders can slide on the shafts. The sliders and shafts are put around the penis, to form the closed circle.

Preferably, the frame and slider forms a main part of the penis enlargement exerciser. This main part is set on a guide rail of a main frame. The guide rail is parallel to the axis of the penis. There is an electromotor set on the main frame or the main part. The electromotor can drive the main part to move back and forth on the guide rail through a transmission system.

Preferably, there are a handle on the slider (or each of the sliders). A user can pull the handles close to each other, to make the sliders hold the penis tightly. This makes it easier to press the penis with hands.

Preferably, the diameter of a portion of the penis protecting cover close to the root of penis is tapered and enlarged along a direction away from the root portion, so that the penis protecting cover can fit with penis of different sizes by cutting off the part of the penis protecting cover whose diameter is smaller than that of the penis. To carry the same purpose, the penis protecting cover has a gap near the root of penis. The gap of the penis protecting cover should be placed on the position misaligned with the gap between the sliders and the frame.

Preferably, to supply the slider with elastic force instead of the force of hand, a spring is linked to the slider and the frame. So the slider can be pushed to press the penis automatically. An adjustment part can be added to adjust the position of the spring to control the value of the elastic force.

The penis protecting cover and the pubic hair separating plate can be integrated into one piece.

Before the penis protecting cover is used, lubrication oil should be used to cover the penis. Then, the penis is enclosed in the penis protecting cover. Two back plates are added to the penis protecting cover, for enlarging the friction force between the protecting cover and the slider to keep the penis protecting cover and the penis enlargement exerciser together. The penis protecting cover may be formed with holes or be cut into several sections to save the material. Two separating plates are disposed at the both side of the touching part between the protecting cover and the penis enlargement exerciser and hold the penis enlargement exerciser, to prevent the separation between the penis protecting cover and the penis enlargement exerciser when using, or tie up the penis protecting cover on the penis enlargement exerciser by a rope through a hole on the penis protecting cover.

A penis enlargement exercising system can provide a plurality of the penis enlargement exerciser with various sizes, which can be supplied to various users to suit each person's penis with different size, and one person can select to use one of the penis enlargement exerciser which the size of his penis varies in the different exercising stage.

When using, to accelerate the blood circulation in the penis, the penis is put inside a penis heating device to be warmed by the far infrared ray for a few minutes. Then, the pubic hair separating plate is put around the root of the penis.

The user can adjust the erection state of his penis according to his requirement, to reach hard erection or half hard erection status (hard erection of penis is required to gain growth of length, and half hard erection is required to gain growth of circumference). Then, the lubrication oil and the penis protecting cover are used onto the penis, and put them into the closed circle of the penis enlargement exerciser. Move the sliders to narrow the closed circle to hold the penis tightly. Then, the inner wall of the closed circle is pushed back and forth on the penis along the direction of the penis axis. The closed circle will push the blood in the penis toward the glans or the root of the penis. During these movements, the volume of the front part on the penis in the movement direction is pressed. The increased blood pressure will make the cavernosa swell. Frequent exercises and proper nutrition supply will make the blood containers of the penis become bigger. And, the penis will become bigger, longer and thicker. The effect has been proven by experiments. When doing reciprocating movements, rubber bands can be hooped around the root of the penis to stop the blood from flowing out of the penis.

Compared to other traditional penis enlargement devices on the market, the advantage of this device is based on completely different theory instead of the simple stretching theory. Frequent exercises of pressing the blood to swell the cavernosa and proper nutrition supply bring a permanent penis enlargement result. It can also improve blood circulation of the penis and improve the erection state of the penis. Frequent exercises will also reduce sensitivity of the penis and it will prolong the sexual intercourse time. Compared to China patent application No.2007101864311 of the applicant of the present application, which has an expandable interspace, but the present invention has a closed circle around the penis and the penis protecting cover to protect the penis, for overcoming the shortcoming of clamping the prepuce.

The U.S. Patent application US2009/0247377 has a part that can slide on a frame to stretch the penis, but can't form a closed circle to press the penis. And, the plane formed by the sliding part and the frame is not vertical to the axis of the penis. These designs are completely different from the present invention.

The structure of the penis protecting layer is simple, liking a condom in the market, but it is specially designed to solve the problem of the new penis enlargement exerciser according to its unique structure. It is a completely new application in this area, and should be novelty and creative. It can also be combined with another production disclosed by the applicant's Chinese patent No. 2007101864311, to avoid the design drawback of the prior art.

### DESCRIPTION OF THE DRAWINGS

The following text will take a description with reference to the accompanying drawings and embodiments, as follows:
FIGS. 1, 2 and 3 are front, left, and top views of a first embodiment of a penis enlargement exercising system of the present invention.
FIG.4 is a perspective view of the first embodiment of the penis enlargement exercising system.
FIG.5 is a perspective view of the first embodiment of the penis enlargement exercising system when it is used on a penis.
FIG.6 is a perspective view of the second embodiment of a penis protecting cover of the present invention.
FIG.7 is a perspective view of a third embodiment of a penis enlargement exerciser.
FIG.8 is a front perspective view of a fourth embodiment of a penis enlargement exerciser.
FIG.9 is a rear perspective view of the fourth embodiment of the penis enlargement exerciser.
FIG.10 is a perspective view of a fifth embodiment of a penis enlargement exerciser.
FIG.11 is a perspective view of the sixth embodiment of a penis protecting cover.
FIG.12 is a perspective view of the seventh embodiment of a pubic hair separating plate of the present invention.
FIG.13 is a perspective view of an eighth embodiment of a penis enlargement exerciser.

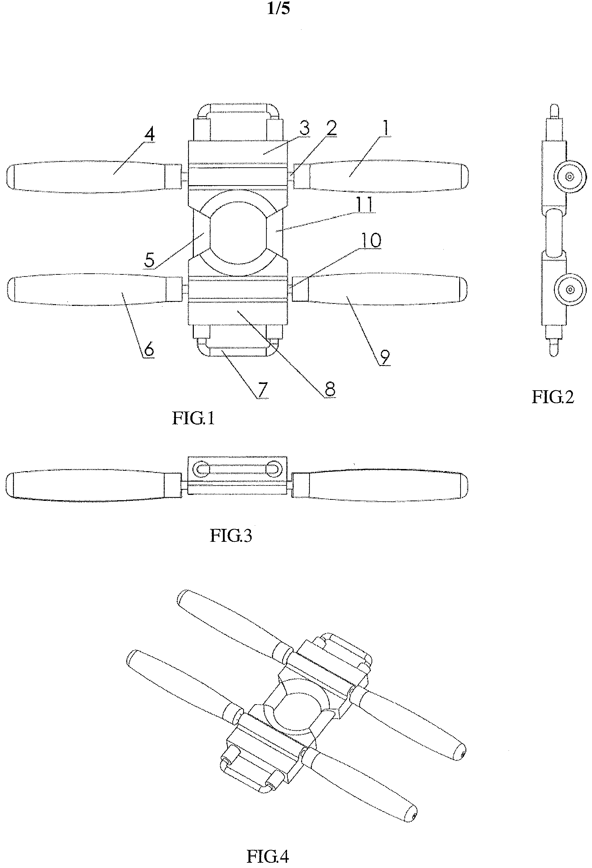

In FIG.1: 1 handle; 2 handle shaft; 3 slider; 4 handle; 5 shaft; 6 handle; 7 rack; 8 slider; 9 handle; 10 handle shaft; 11 shaft;

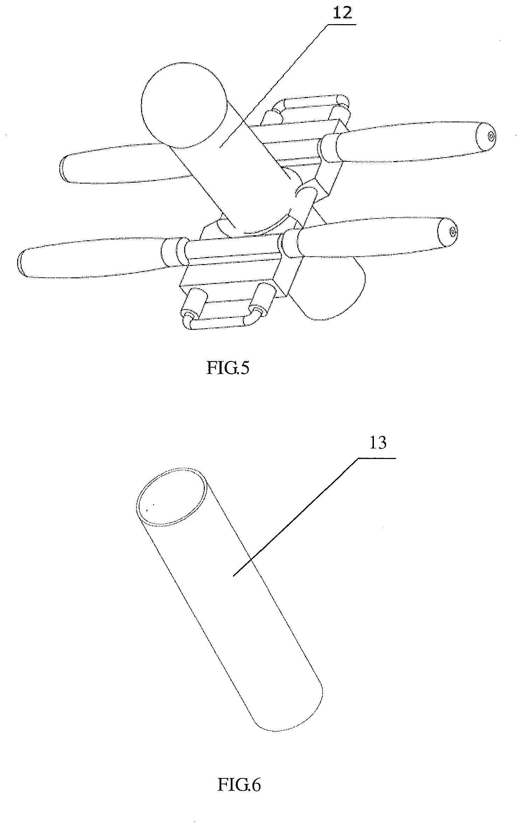

In FIG.5: 12 penis;
In FIG.6: 13 penis protecting layer;

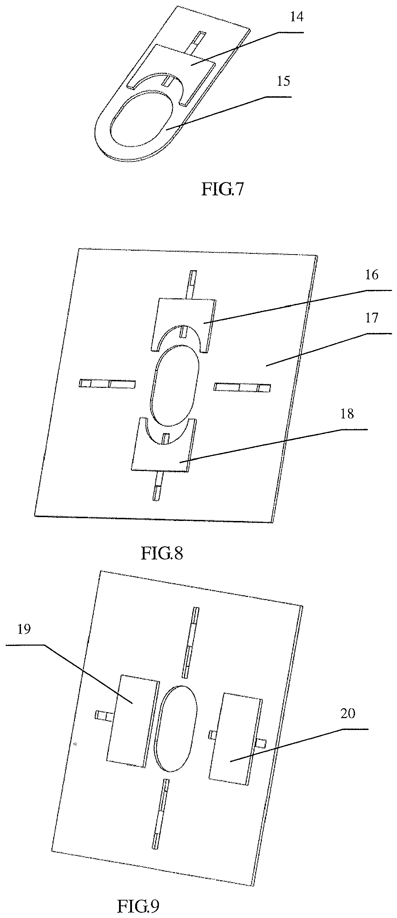

In FIG.7: 14 slider; 15 frame;
In FIG.8: 16 slider; 17 frame; 18 slider;
In FIG.9: 19 slider; 20 slider;

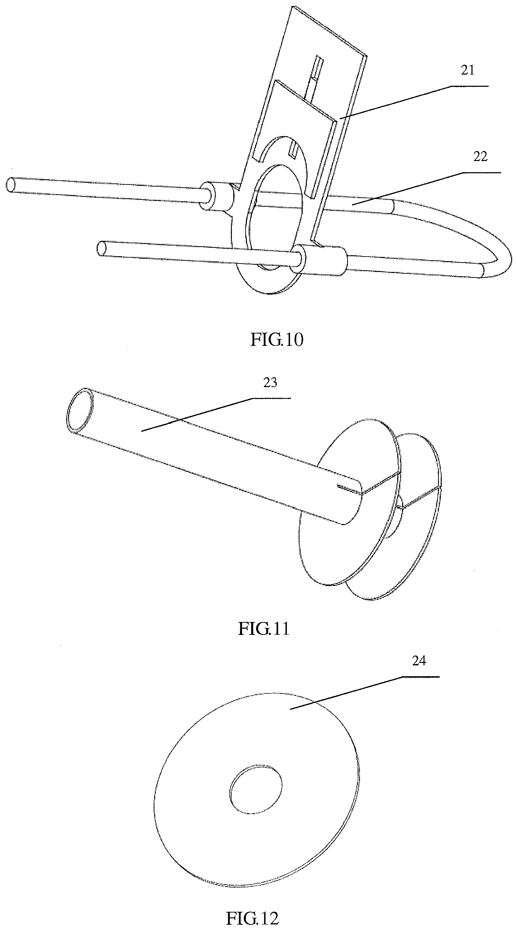

In FIG.10: 21 main part; 22 guide rail;
In FIG.11: 23 penis protecting layer;
In FIG.12: 24 pubic hair separating plate;

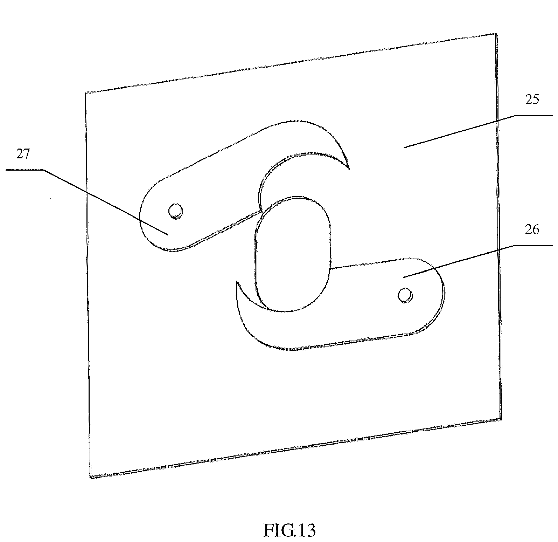

In FIG.13: 25 frame; 26 slider; 27 slider

### DETAILED DESCRIPTION OF THE PREFERRED EMBODIMENTS

The structure and the technical means adopted by the present invention to achieve the above and other objects can be best understood by referring to the following detailed description of the preferred embodiments and the accompanying drawings. Furthermore, directional terms described by the present invention, such as upper, lower, front, back, left, right, inner, outer, side, longitudinal/vertical, transverse/horizontal, and etc., are only directions by referring to the accompanying drawings, and thus the used directional terms are used to describe and understand the present invention, but the present invention is not limited thereto.

The first embodiment is an embodiment of a penis enlargement exerciser of the present invention. In FIG.1, a rack 7 is set in axle holes of shafts 5 and 11, both of which are hollow, and then the rack 7 and the shafts 5, 11 commonly form a frame. The shafts 5 and 11 are made of plastic, and the rack 7 is made of stainless steel for the cost down. Ends of two handle shafts 2, 10 are inserted into axle holes of handles 1, 4, 6 and 9. Middle portions of the handle shaft 2, 10 are inserted in the holes on two sliders 3, 8. The shafts 5, 11 pass through holes in the slider 3, 8. The sliders 3, 8 can slide on the two shafts 5, 11.

The positions of the penis and the penis enlargement exerciser in use are shown in FIG. 5. The penis 12 is firstly covered with lubrication oil. And, a penis protecting cover 13 shown in FIG. 6 will be set on the penis. Then, the penis with the penis protecting cover 13 is put in the closed circle (hole) which is formed by the shafts 5, 11 and the sliders 3, 8. When the user pushes the handles 1, 4 toward the handles 6, 9, the sliders 3, 8 will slide toward each other on the shafts 5, 11. Then, the penis will be held tightly in the closed circle. Then, the penis enlargement exerciser will be pushed back and forth on the penis repeatedly.

The second embodiment as shown in FIG. 6 is the simplest embodiment of the penis protecting cover 13 of the present invention.

The third embodiment in FIG.7 is an embodiment of the penis enlargement exerciser in the penis enlargement exercising system. A slider 14 can slide in the groove of a frame 15. A curved edge of the slider 14 and a circle or oval-shaped hole of the frame 15 commonly form a closed circle. When the slider 14 is pushed down, the penis will be held tightly by inner edges of the closed circle.

Four sliders 16, 18, 19, 20 are movably disposed on a frame 17 in the forth embodiment shown in FIG.8 and FIG.9. Two of the sliders 16, 18 are disposed on one side of the frame 17, and the other sliders 19,20 are disposed on the other side of the frame 17. The sliders 16, 18, 19, 20 can slide in the corresponding grooves of the frame 17 to commonly form a closed circle, and the closed circle can hold the penis tightly from four sides of the frame 17.

A main part 21 can slide back and forth on a guide rail 22 in the fifth embodiment shown in FIG.10. To simplify the drawing, the electromotor, gearing, and control unit, etc, are omitted herein.

The sixth embodiment in FIG.11 is an embodiment of a penis protecting cover 23. The penis protecting cover 23 can be made of silicon gel, rubber or latex. Two ring-like plates are movably disposed on the penis protecting cover 23, and are used to fix the position of the penis protecting cover 23 on the penis enlargement exerciser. A slit is formed on the root of the ring-like plates and the penis protecting cover 23, and thus the penis protecting cover 23 is possible to suit various penises of different sizes.

The seventh embodiment in FIG.12 is an embodiment of a pubic hair separating plate 24 of the present invention. It can be made of silicon gel, rubber or latex. The penis is put in the hole on the pubic hair separating plate 24. The pubic hair separating plate 24 is put between the pubic hair at the root of the penis and the penis enlargement exerciser. When the penis grows thicker, the hole on the pubic hair separating plate 24 can be cut wider to hold the penis.

The eighth embodiment in FIG.13 is an embodiment of the penis enlargement exerciser in the system. Two sliders 26, 27 can rotate round two pivotal shafts on a frame 25. When the sliders 26, 27 are pushed toward the hole of the frame 25 to commonly form a closed circle, the penis will be held tightly by the closed circle. There are only two sliders 26, 27 shown in this figure. In actual product, there can be more sliders on the frame 25. To simplify the drawing, the forcing unit is omitted herein.

For the embodiments above, the parts which are in contact with the penis can have smooth curved surfaces to make it easier to move on the prepuce of the penis. To simplify the drawing, the structure of smooth curved surfaces is not drawn. For some embodiments, handles are also omitted.

The present invention has been described with a preferred embodiment thereof and it is understood that many changes and modifications to the described embodiment can be carried out without departing from the scope of the invention that is intended to be limited only by the appended claims.

## Claims

1. A penis enlargement exercising system comprising a penis enlargement exerciser having a rigid frame (5, 7, 11, 15, 17, 25) the system **characterized in that** the penis enlargement exerciser further comprises at least one slider (3, 8, 14, 16, 18, 19, 20, 26, 27); wherein the slider (3, 8, 14, 16, 18, 19, 20, 26, 27) can slide on the frame; wherein the frame (5, 7, 11, 15, 17, 25) and the slider (3, 8, 14, 16, 18, 19, 20, 26, 27) commonly form a closed circle around a penis (12) of a user; wherein the closed circle is similar to the cross-section of the penis (12); wherein the closed circle has no gap in its circumferential direction; wherein when using the penis enlargement exerciser, the penis (12) can be put into the closed circle, so that the longitudinal axis of the penis is substantially perpendicular to the closed circle, the slider (3, 8, 14, 16, 18, 19, 20, 26, 27) can be forced to move toward the longitudinal axis of the penis (12) to push the slider (3, 8, 14, 16, 18, 19, 20, 26, 27) to the center of the closed circle, so that the closed circle is narrowed and tightly kept around the penis, thereby causing the penis (12) to be held tightly by the slider (3, 8, 14, 16, 18, 19, 20, 26, 27) and the frame (5, 7, 11, 15, 17, 25), wherein the closed circle can slide back and forth on the penis (12) in the direction of the longitudinal axis thereof, so that the blood inside the penis (12) can be pressed to enlarge a front part of the cavernous body of the penis (12) in a moving direction of the penis enlargement exerciser.

2. The penis enlargement exercising system according to claim 1, **characterized in that** the penis enlargement exercising system further comprises a penis heating device which is tube shaped or half-tube shaped and has a cross-section area slightly greater than that of the penis; wherein the penis heating element is made of material which generates far infrared rays, and includes a heating control circuit and components thereof; when using the penis enlargement exerciser, the penis is put into the penis heating device which connects with a power source, and then the far infrared rays radiated by the penis heating device covers and warms up the penis.

3. The penis enlargement exercising system according to claim 1, **characterized in that** the frame (5, 7, 11, 15, 17, 25) has a rigid U-shaped part (22) which has two shafts parallel to each other; two holes parallel to each other are formed on the slider (3, 8, 14, 16, 18, 19, 20, 26, 27); and the two shafts pass through the two holes, respectively.

4. The penis enlargement exercising system according to claim 1, **characterized in that** the penis enlargement exercising system includes two sliders (3, 8); the frame (5, 7, 11) has at least two shafts (5, 11) parallel to each other; two holes parallel to each other are formed on the sliders (3, 8); the two shafts (5, 11) pass through the two holes, respectively; and the sliders (3, 8) slide on the shafts.

5. The penis enlargement exercising system according to claim 1, **characterized in that** the penis enlargement exerciser has at least two of the sliders (3, 8, 16, 18, 19, 20, 26, 27); and the sliders (3, 8, 16, 18, 19, 20, 26, 27) are disposed around the penis (12).

6. The penis enlargement exercising system according to claim 1, **characterized in that** the penis enlargement exerciser has at least two of the sliders (3, 8); two holes parallel to each other are formed on each of the sliders (3, 8); the frame (5, 7, 11) has at least two shafts (5, 11) parallel to each other; the shafts (5, 11) pass through the holes on the sliders (3, 8), respectively; the sliders (3, 8) slide on the shafts (5, 11); and the sliders (3, 8) and shafts (5, 11) are disposed around the penis, to form the closed circle.

7. The penis enlargement exercising system according to claim 1, **characterized in that** the sliders (3, 8, 14, 16, 18, 19, 20, 26, 27) slide or rotate around an axis of the frame.

8. The penis enlargement exercising system according to claim 1, **characterized in that** the frame (5, 7, 11, 15, 17, 25) and the slider (3, 8, 14, 16, 18, 19, 20, 26, 27) form a main part of the penis enlargement exerciser; the main part is installed on a guide rail (22) of a main frame; and the guide rail (22) is parallel to the axis of the penis; wherein an electromotor is mounted on the main frame or the main part; and the electromotor drives the main part to move on the guide rail (22) through a transmission system.

9. The penis enlargement exercising system according to claim 1, **characterized in that** the penis enlargement exercising system further comprises a penis protecting layer or cover (13, 23), which is tube shaped or half tube shaped and is made of soft material, to protect the skin of the penis; wherein the penis protecting layer or cover (13, 23) is disposed around the penis and separates the penis from the gap between the slider and the frame.

10. The penis enlargement exercising system according to claim 1, **characterized in that** a handle (1, 4, 6, 9) is formed on the slider (3, 8).

11. The penis enlargement exercising system according to claim 1, **characterized in that** the sliders (16, 18, 19, 20) are movably disposed on the frame (17) to commonly form the closed circle.

## Patentansprüche

1. Trainingssystem zur Vergrößerung des Penis, umfassend
ein Penisvergrößerungs-Trainingsgerät mit einem starren Rahmen (5, 7, 11, 15, 17, 25), wobei das System **dadurch gekennzeichnet ist, dass** das Penisvergrößerungs-Trainingsgerät ferner zumindest einen Schieber (3, 8, 14, 16, 18, 19, 20, 26, 27) umfasst; worin der Schieber (3, 8, 14, 16, 18, 19, 20, 26, 27) an dem Rahmen gleiten kann; worin der Rahmen (5, 7, 11, 15, 17, 25) und der Schieber (3, 8, 14, 16, 18, 19, 20, 26, 27) in üblicher Weise einen geschlossenen Kreis um den Penis (12) eines Benutzers herum bilden;
worin der geschlossene Kreis dem Querschnitt des Penis (12) ähnlich ist;
worin der geschlossene Kreis keinen Spalt in seiner Umfangsrichtung hat; worin bei der Benutzung des Penisvergrößerungs-Trainingsgeräts der Penis (12) in den geschlossenen Kreis hinein gesteckt werden kann, so dass die Längsachse des Penis zum geschlossenen Kreis im Wesentlichen senkrecht ist, der Schieber (3, 8, 14, 16, 18, 19, 20, 26, 27) dazu gezwungen werden kann, sich in Richtung auf die Längsachse des Penis (12) zu bewegen, um den Schieber (3, 8, 14, 16, 18, 19, 20, 26, 27) zum Mittelpunkt des geschlossenen Kreises zu schieben, so dass der geschlossene Kreis verengt und fest um den Penis gehalten wird, wodurch der Penis (12) durch den Schieber (3, 8, 14, 16, 18, 19, 20, 26, 27) und den Rahmen (5, 7, 11, 15, 17, 25) eng gehalten wird, worin der geschlossene Kreis am Penis (12) in der Richtung seiner Längsachse rückwärts und vorwärts gleiten kann, so dass das Blut innerhalb des Penis (12) gepresst werden kann, um einen Vorderteil des Schwellkörpers des Penis (12) in einer Bewegungsrichtung des Penisvergrößerungs-Trainingsgeräts zu vergrößern.

2. Trainingssystem zur Vergrößerung des Penis nach Anspruch 1, **dadurch gekennzeichnet, dass** das Trainingssystem zur Vergrößerung des Penis ferner eine Penisheizvorrichtung umfasst, die rohrförmig oder halbrohrförmig ist und eine Querschnittsfläche hat, die etwas größer als diejenige des Penis ist; worin das Penisheizelement aus einem Material besteht, das ferne Infrarotstrahlen erzeugt und eine Heizsteuerschaltung und Komponenten davon umfasst; wenn das Penisvergrößerungs-Trainingsgerät benutzt wird, der Penis in die Penisheizvorrichtung hinein gesteckt wird, die mit einer Stromquelle verbunden wird, und dann die fernen Infrarotstrahlen, die von der Penisheizvorrichtung ausgestrahlt werden, den Penis decken und erwärmen.

3. Trainingssystem zur Vergrößerung des Penis nach Anspruch 1, **dadurch gekennzeichnet, dass** der Rahmen (5, 7, 11, 15, 17, 25) einen starren U-förmigen Teil (22) hat, der zwei zueinander parallele Schäfte aufweist; zwei zueinander parallele Löcher am Schieber (3, 8, 14, 16, 18, 19, 20, 26, 27) geformt sind; und die zwei Schäfte jeweils die zwei Löcher durchlaufen.

4. Trainingssystem zur Vergrößerung des Penis nach Anspruch 1, **dadurch gekennzeichnet, dass** das Trainingssystem zur Vergrößerung des Penis zwei Schieber (3, 8) umfasst; der Rahmen (5, 7, 11) zumindest zwei Schäfte (5, 11) hat, die parallel zueinander sind; zwei Löcher, die parallel zueinander sind, an den Schiebern (3, 8) geformt sind; die zwei Schäfte (5, 11) jeweils die zwei Löcher durchlaufen; und die Schieber (3, 8) an den Schäften gleiten.

5. Trainingssystem zur Vergrößerung des Penis nach Anspruch 1, **dadurch gekennzeichnet, dass** das Penisvergrößerungs-Trainingsgerät zumindest zwei von den Schiebern (3, 8, 16, 18, 19, 20, 26, 27) aufweist; und die Schieber (3, 8, 16, 18, 19, 20, 26, 27) um den Penis (12) angeordnet sind.

6. Trainingssystem zur Vergrößerung des Penis nach Anspruch 1, **dadurch gekennzeichnet, dass** das Penisvergrößerungs-Trainingsgerät zumindest zwei von den Schiebern (3, 8) aufweist; zwei zueinander parallele Löcher an jedem der Schieber (3, 8) geformt sind; der Rahmen (5, 7, 11) zumindest zwei Schäfte (5, 11) aufweist, die parallel zueinander sind; die Schäfte (5, 11) jeweils die Löcher an den Schiebern (3, 8) durchlaufen; die Schieber (3, 8) an den Schäften (5, 11) gleiten; und die Schieber (3, 8) und die Schäfte (5, 11) um den Penis angeordnet sind, um den geschlossenen Kreis zu bilden.

7. Trainingssystem zur Vergrößerung des Penis nach Anspruch 1, **dadurch gekennzeichnet, dass** die Schieber (3, 8, 14, 16, 18, 19, 20, 26, 27) um eine Achse des Rahmens gleiten oder rotieren.

8. Trainingssystem zur Vergrößerung des Penis nach Anspruch 1, **dadurch gekennzeichnet, dass** der Rahmen (5, 7, 11, 15, 17, 25) und der Schieber (3, 8, 14, 16, 18, 19, 20, 26, 27) einen Hauptteil des Penisvergrößerungs-Trainingsgeräts bilden; der Hauptteil an einer Führungsschiene (22) eines Hauptrahmens installiert ist; und die Führungsschiene (22) zur Achse des Penis parallel ist; worin ein Elektromotor am Hauptrahmen oder am Hauptteil montiert ist; und der Elektromotor den Hauptteil ansteuert, damit dieser sich an der Führungsschiene (22) über ein Übertragungssystem bewegt.

9. Trainingssystem zur Vergrößerung des Penis nach Anspruch 1, **dadurch gekennzeichnet, dass** das Trainingssystem zur Vergrößerung des Penis ferner eine Penisschutzschicht oder -abdeckung (13, 23) umfasst, die rohrförmig oder halbrohrförmig ist und aus einem weichen Material besteht, um die Penishaut zu schützen; worin die Penisschutzschicht oder -abdeckung (13, 23) um den Penis angeordnet ist und den Penis von dem Spalt zwischen dem Schieber und dem Rahmen trennt.

10. Trainingssystem zur Vergrößerung des Penis nach Anspruch 1, **dadurch gekennzeichnet, dass** ein Handgriff (1, 4, 6, 9) an dem Schieber (3, 8) geformt ist.

11. Trainingssystem zur Vergrößerung des Penis nach Anspruch 1, **dadurch gekennzeichnet, dass** die Schieber (16, 18, 19, 20) am Rahmen (17) beweglich angeordnet sind, um den geschlossenen Kreis in üblicher Weise zu bilden.

## Revendications

1. Système d'exercice d'agrandissement du pénis comprenant un appareil d'exercice d'agrandissement du pénis ayant un cadre rigide (5, 7, 11, 15, 17, 25), le système étant **caractérisé en ce que** l'appareil d'exercice d'agrandissement du pénis comprend en outre au moins un dispositif coulissant (3, 8, 14, 16 , 18, 19, 20, 26, 27) ; où le dispositif coulissant (3, 8, 14, 16, 18, 19, 20, 26, 27) peut coulisser sur le cadre ; où le cadre (5, 7, 11, 15, 17, 25) et le dispositif coulissant (3, 8, 14, 16, 18, 19, 20, 26, 27) forment communément un cercle fermé autour d'un pénis (12) d'un utilisateur ; où le cercle fermé est similaire à la section transversale du pénis (12) ; où le cercle fermé n'a pas d'espace dans sa direction circonférentielle ; où lors de l'utilisation de l'appareil d'exercice d'agrandissement du pénis, le pénis (12) peut être placé dans le cercle fermé, de sorte que l'axe longitudinal du pénis soit substantiellement perpendiculaire au cercle fermé, le dispositif coulissant (3, 8, 14, 16, 18, 19, 20, 26, 27) peut être forcé de se déplacer vers l'axe longitudinal du pénis (12) pour pousser le dispositif coulissant (3, 8, 14, 16, 18, 19, 20, 26, 27) vers le centre de le cercle fermé, de sorte que le cercle fermé soit rétréci et maintenu fermement autour du pénis, ce qui oblige le pénis (12) à être maintenu fermement par le dispositif coulissant (3, 8, 14, 16, 18, 19, 20, 26, 27) et le cadre (5, 7, 11, 15, 17, 25), où le cercle fermé peut coulisser en avant et en arrière sur le pénis (12) dans la direction de son axe longitudinal, de sorte que le sang à l'intérieur du pénis (12) puisse être pressé pour agrandir une partie avant des corps caverneux du pénis (12) dans une direction de déplacement de l'appareil d'exercice d'agrandissement du pénis.

2. Système d'exercice d'agrandissement du pénis selon la revendication 1, **caractérisé en ce que** le système d'exercice d'agrandissement du pénis comprend en outre un dispositif de chauffage du pénis qui est en forme de tube ou en demi-tube et qui a une section transversale légèrement supérieure à celle du pénis ; où l'élément de chauffage du pénis est réalisé avec un matériau qui génère des rayons infrarouges lointains, et comprend un circuit de commande de chauffage et ses composants ; lors de l'utilisation de l'appareil d'exercice d'agrandissement du pénis, le pénis est placé dans le dispositif de chauffage du pénis qui se connecte à une source d'alimentation, puis les rayons infrarouges lointains rayonnés par le dispositif de chauffage du pénis couvrent et réchauffent le pénis.

3. Système d'exercice d'agrandissement du pénis selon la revendication 1, **caractérisé en ce que** le cadre (5, 7, 11, 15, 17, 25) a une partie rigide en forme de U (22) qui a deux tiges parallèles entre elles ; deux trous parallèles l'un à l'autre sont formés sur le dispositif coulissant (3, 8, 14, 16, 18, 19, 20, 26, 27) ; et les deux tiges traversent respectivement les deux trous.

4. Système d'exercice d'agrandissement du pénis selon la revendication 1, **caractérisé en ce que** le système d'exercice d'agrandissement du pénis comprend deux dispositifs coulissants (3, 8) ; le cadre (5, 7, 11) a au moins deux tiges (5, 11) parallèles entre elles ; deux trous parallèles l'un à l'autre sont formés sur les dispositifs coulissants (3, 8) ; les deux tiges (5, 11) traversent respectivement les deux trous ; et les dispositifs coulissants (3, 8) coulissent sur les tiges.

5. Système d'exercice d'agrandissement du pénis selon la revendication 1, **caractérisé en ce que** l'appareil d'exercice d'agrandissement du pénis a au moins deux des dispositifs coulissants (3, 8, 16, 18, 19, 20, 26, 27) ; et les dispositifs coulissants (3, 8, 16, 18, 19, 20, 26, 27) sont disposés autour du pénis (12).

6. Système d'exercice d'agrandissement du pénis selon la revendication 1, **caractérisé en ce que** l'appareil d'exercice d'agrandissement du pénis a au moins deux des dispositifs coulissants (3, 8) ; deux trous parallèles l'un à l'autre sont formés sur chacun des dispositifs coulissants (3, 8) ; le cadre (5, 7, 11) a au moins deux tiges (5, 11) parallèles entre elles ; les tiges (5, 11) traversent respectivement les trous sur le dispositifs coulissants (3, 8) ; les dispositifs coulissants (3, 8) coulissent sur les tiges (5, 11) ; et les dispositifs coulissants (3, 8) et les tiges (5, 11) sont disposés autour du pénis, pour former le cercle fermé.

7. Système d'exercice d'agrandissement du pénis selon la revendication 1, **caractérisé en ce que** les dispositifs coulissants (3, 8, 14, 16, 18, 19, 20, 26, 27) coulissent ou tournent autour d'un axe du cadre.

8. Système d'exercice d'agrandissement du pénis selon la revendication 1, **caractérisé en ce que** le cadre (5, 7, 11, 15, 17, 25) et le dispositif coulissant (3, 8, 14, 16, 18, 19, 20, 26, 27) forment une partie principale de l'appareil d'exercice d'agrandissement du pénis ; la partie principale est installée sur un rail de guidage (22) d'un cadre principal ; et le rail de guidage (22) est parallèle à l'axe du pénis ; où un électromoteur est monté sur le cadre principal ou la partie principale ; et l'électromoteur entraîne la partie principale à se déplacer sur le rail de guidage (22) à travers un système de transmission.

9. Système d'exercice d'agrandissement du pénis selon la revendication 1, **caractérisé en ce que** le système d'exercice d'agrandissement du pénis comprend en outre une couche ou une couverture de protection (13, 23) du pénis, qui est en forme de tube ou en forme de demi-tube et qui est réalisée avec un matériau souple, pour protéger la peau du pénis ; où la couche ou la couverture de protection (13, 23) du pénis est disposée autour du pénis et sépare le pénis de l'espace entre le dispositif coulissant et le cadre.

10. Système d'exercice d'agrandissement du pénis selon la revendication 1, **caractérisé en ce qu'**une poignée (1, 4, 6, 9) est formée sur le dispositif coulissant (3, 8).

11. Système d'exercice d'agrandissement du pénis selon la revendication 1, **caractérisé en ce que** les dispositifs coulissants (16, 18, 19, 20) sont disposés de manière mobile sur le cadre (17) pour former communément le cercle fermé.
